# EUROPÄISCHE PATENTSCHRIFT

(11) **EP 1 856 021 B1**
(45) Veröffentlichungstag und Bekanntmachung des Hinweises auf die Patenterteilung: **05.08.2015**
(21) Anmeldenummer: 06708585.2
(22) Anmeldetag: 01.03.2006
(51) Int. Cl.: C07C 51/43

(54) **VERFAHREN ZUR ABTRENNUNG VON METHACROLEIN AUS ACRYLSÄURE ALS HAUPTBESTANDTEIL SOWIE ZIELPRODUKT UND METHACROLEIN ALS NEBENKOMPONENTE ENTHALTENDER FLÜSSIGER PHASE**
METHOD FOR SEPARATING METHACROLEIN FROM ACRYLIC ACID AS THE MAIN CONSTITUENT AND FROM A LIQUID PHASE CONTAINING METHACROLEIN AS A MINOR CONSTITUENT, IN ADDITION TO A TARGET PRODUCT
PROCEDE PERMETTANT DE SEPARER L'ALDEHYDE METHACRYLIQUE DE L'ACIDE ACRYLIQUE EN TANT QUE CONSTITUANT PRINCIPAL, ET PHASE LIQUIDE CONTENANT UN PRODUIT CIBLE ET L'ALDEHYDE METHACRYLIQUE EN TANT DE CONSTITUANTS SECONDAIRES

(30) Priorität: 01.03.2005 DE 102005009887; 01.03.2005 US 656882 P; 05.04.2005 DE 102005015637; 05.04.2005 US 668088 P
(43) Veröffentlichungstag der Anmeldung: 21.11.2007
(73) Patentinhaber: BASF SE, 67056 Ludwigshafen (DE)
(72) Erfinder: HEILEK, Jörg, 69245 Bammental (DE); MÜLLER-ENGEL, Klaus Joachim, 76297 Stutensee (DE); ADAMI, Christoph, 69469 Weinheim (DE); DIETERLE, Martin, 68167 Mannheim (DE)
(86) Internationale Anmeldenummer: PCT/EP2006/060366
(87) Internationale Veröffentlichungsnummer: WO 2006/092415

(56) Entgegenhaltungen:
- WO-A-01/92197
- DE-A1- 19 627 847
- US-A1- 2003 060 661

## Beschreibung

Vorliegende Erfindung betrifft ein Verfahren zur Abtrennung von Methacrolein aus Acrylsäure als Hauptbestandteil sowie Zielprodukt und Methacrolein als Nebenkomponente enthaltender flüssiger Phase.

Acrylsäure ist ein bedeutendes Monomeres, das als solches oder in Form seiner Alkylester zur Erzeugung von z. B. als Klebstoffe geeigneten oder Wasser superabsorbierenden Polymerisaten Verwendung findet (vgl. z. B. WO 02/055469 und WO 03/078378).

Die großtechnische Herstellung von Acrylsäure erfolgt weltweit nahezu ausschließlich nach dem Verfahren der (in der Regel zweistufigen) heterogen katalysierten Partialoxidation von Propylen (vgl. z. B. EP-A 990 636, US-A 5,198,578, EP-A 10 15 410, EP-A 14 84 303, EP-A 14 84 308, EP-A 14 84 309 und US-A 2004/0242826). Als Ausgangspropylen wird dabei Propylen von vergleichsweise hoher Reinheit verwendet (vgl. DE-A 1Q1 31 297). Die Erzeugung von solchermaßen reinem Roh-Propylen ist relativ aufwendig und kostspielig. Sie geht normalerweise von rohen paraffinischen Kohlenwasserstoffen aus und beinhaltet in der Regel verschiedene Reinigungsstufen, um das gebildete Propylen hochrein zu isolieren (vgl. DE-A 35 21 458). Diese Reinigungsstufen umfassen in der Regel Trennungen von von Propylen verschiedenen Olefinen sowie von von Propylen verschiedenen anderen Nebenprodukten, einschließlich der im rohen paraffinischen Kohlenwasserstoff bereits enthaltenen Nebenkomponenten.

Von Bedeutung ist dabei insbesondere die Trennung des Propylen von seinem Begleiter Propan. Infolge der physikalischen Ähnlichkeit beider Verbindungen ist vor allem diese Abtrennung investitions- und energieintensiv. Da die überwiegende Menge des so gewonnenen Roh-Propylen in großen Mengen für nachfolgende Polymerisationen (z. B. zur Herstellung von Polypropylen) verwendet wird, (wo die beschriebene hohe Reinheit unabdingbar ist) und dabei eine hohe Wertschöpfung erfährt, sind vorgenannte Abtrennungen trotz des damit einhergehenden Aufwandes im Verbund mit Raffineriecrackern und Steamcrackern üblich und bilden großtechnisch den Stand der Technik. Der von diesen Roh-Propylenen in die Partialoxidation zu Acrylsäure fließende Anteil ist gegenüber dem Bedarf für Polypropylen von eher untergeordneter Bedeutung und läuft als Nebenbedarfsstrom zu noch akzeptablen Rohstoffpreisen mit.

Charakteristisch für die Herstellung von Acrylsäure durch gasphasenkatalytische Partialoxidation von solchermaßen vergleichsweise reinem Propylen ist, dass die Acrylsäure trotz der verfügbaren Reinheit des Rohstoffs dabei nicht als solche, sondern insbesondere infolge von parallel erfolgenden Nebenreaktionen, als Bestandteil eines Produktgasgemischs erhalten wird, aus welchem sie nachfolgend abgetrennt werden muss.

Dieses Produktgasgemisch enthält in der Regel auch nicht vollständig umgesetzte Reaktanden, gegebenenfalls nicht vollständig umgesetzte Zwischenprodukte sowie aus Gründen der verbesserten Wärmeabfuhr sowie zur Gewährleistung eines nicht explosiven Verhaltens mitverwendetes inertes Verdünnungsgas.

Als ein inertes Verdünnungsgas soll in dieser Schrift ein Reaktionsgasbestandteil verstanden werden, der sich unter den Bedingungen der relevanten Reaktion als inert verhält und - jeder inerte Reaktionsgasbestandteil für sich betrachtet - zu mehr als 95 mol-%, vorzugsweise zu mehr als 97, oder 98 oder 99 mol-% chemisch unverändert erhalten bleibt.

Im wesentlichen allen diesbezüglich bekannten Trennverfahren ist gemein, dass, gegebenenfalls nach direktem und/oder indirektem Abkühlen des vorgenannten Produktgasgemischs, im Produktgasgemisch enthaltene Acrylsäure in einem Grundabtrennschritt in die kondensierte (insbesondere flüssige) Phase überführt wird.

Dies kann z. B. durch Absorption in ein geeignetes Lösungsmittel (z. B. Wasser, hochsiedende organische Lösungsmittel, wässrige Lösungen) und/oder durch partielle oder im wesentlichen vollständige Kondensation (z. B. fraktionierende Kondensation) erfolgen (vgl. dazu die eingangs erwähnten Schriften, sowie die Schriften EP-A 13 88 533, EP-A 13 88 532, DE-A 102 35 847, EP-A 79 28 67, WO 98/01415, EP-A 10 15 411, EP-A 10 15 410, WO 99/50219, WO 00/53560, WO 02/09839, DE-A 102 35 847, WO 03/041833, DE-A 102 23 058, DE-A 102 43 625, DE-A 103 36 386, EP-A 85 41 29, US-A 4,317,926, DE-A 198 37 520, DE-A 196 06 877, DE-A 190 50 1325, DE-A 102 47 240, DE-A 197 40 253, EP-A 69 57 36, EP-A 98 22 87, EP-A 10 41 062, EP-A 11 71 46, DE-A 43 08 087, DE-A 43 35 172, DE-A 44 36 243, DE-A 19 924 532, DE-A 103 32 758 sowie DE-A 19 924 533). Eine Acrylsäureabtrennung kann auch wie in der EP-A 98 22 87, der EP-A 98 22 89, der DE-A 103 36 386, der DE-A 101 15 277, der DE-A 196 06 877, der DE-A 197 40 252, der DE-A 196 27 847, der EP-A 92 04 08, der EP-A 10 68 174, der EP-A 10 66 239, der EP-A 10 66 240, der WO 00/53560, der WO 00/53561, der DE-A 100 53 086 und der EP-A 98 22 88 vorgenommen werden. Günstige Abtrennweisen sind auch die in den Schriften WO 2004/063138, WO 2004/035514, DE-A 102 43 625 und DE-A 102 35 847 beschriebenen Verfahren.

Die weitere Abtrennung der Acrylsäure aus den im Rahmen der beschriebenen Grundabtrennung anfallenden, das Zielprodukt Acrylsäure enthaltenden flüssigen (kondensierten) Phasen, wird in den Verfahren des bekannten Standes der Technik je nach den neben Acrylsäure enthaltenen sonstigen, insbesondere von den für die Partialoxidation im einzelnen verwendeten Katalysatoren und sonstigen gewählten Partialoxidationsbedingungen abhängigen, Nebenprodukten durch unterschiedlichste Kombinationen von extraktiven, desorptiven, rektifikativen, azeotrop-destillativen und/oder kristallisativen Verfahren bis zum gewünschten Reinheitsgrad der Acrylsäure vorgenommen. Besonders hohe Reinheitsansprüche werden an die Acrylsäure dann gestellt, wenn sie zur Herstellung von Wasser superabsorbierenden Polymerisaten (Polyacrylsäuren, bzw. Alkalimetallsalze derselben) verwendet werden soll, da solche Polymerisate insbesondere im Hygienebereich Anwendung finden, wo medizinische Standards gelten. Ziel der überwiegenden Anzahl von Acrylsäureherstellungsverfahren ist daher ein möglichst wirtschaftlicher Weg zu solchermaßen superabsorbertauglicher Reinacrylsäure.

Charakteristisch für den beschriebenen konventionellen Acrylsäureherstellweg ist, dass er eine Nebenproduktbildung von Methacrolein allenfalls in analytisch nicht nachweisbaren bzw. in analytisch nicht augenfälligen Mengen beinhaltet, was primär auf den hohen Reinheitsgrad des verwendeten Roh-Propylen zurückzuführen ist. So wird in keiner der nachfolgenden Schriften des Standes der Technik Methacrolein als mögliche Nebenkomponente von Acrylsäure auch nur erwähnt, obgleich die Mehrzahl dieser Schriften detaillierteste Nebenkomponentenanalysen umfasst: WO 98/01414, WO 01/92197, EP-A 648 732, EP-A 1305097, EP-A 14 84 308, EP-A 14 84 309, US-A 2004/0242826, DE-A 103 36 386, WO 02/055496, WO 03/078378, WO 01/77056, WO 03/041833, DE-A 196 06 877, EP-A 792 867, EP-A 920 408, EP-A 10 15 411, EP-A 10 15 410, DE-A 198 38 845, WO 03/041833, WO 02/090310, DE-A 101 22 787, WO 03/041832, EP-A 10 68 174, EP-A 10 66 239, EP-A 11 63 201, EP-A 11 59 249, EP-A 11 89 861, EP-A 12 52 129, WO 01/77056, DE-A 102 35 847, DE-A 102 43 625 und WO 2004/035514. Die gleiche Aussage trifft auf die in den vorgenannten Schriften als jeweiliger Stand der Technik referierten Schriften zu.

Auf der anderen Seite wäre Methacrolein, so es dann als Nebenkomponentbegleiter im Rahmen einer Herstellung von Acrylsäure gebildet würde, ein besonders unangenehmer und im Stand der Technik nicht unerwähnt gebliebener Acrylsäurebegleiter. Dies vor allem deshalb, weil die Neigung von Methacrolein zur radikalischen Polymerisation insbesondere infolge des positiven induktiven Effektes der sie unter anderem von Acrylsäure unterscheidenden Methylgruppe im Vergleich zur selben Neigung der Acrylsäure signifikant gemindert bzw. von selbiger verschieden ist.

D. h., bei der Verwendung von Methacrolein auch nur in Spuren enthaltender Acrylsäure zur Herstellung von Wasser superabsorbierenden radikalisch polymerisierten Polymerisaten, muss davon ausgegangen werden, dass das Methacrolein unter den jeweils gewählten Polymerisationsbedingungen nicht ausreichend einpolymerisiert wird und als vinylisch ungesättigte Verbindung im gebildeten Polymerisat verbleibt, was bei Anwendungen im Hygienebereich problematisch ist. Auch kann ein Beisein von Methacrolein die Polymerisatqualität (z. B. Molekulargewichtsverteilung, Vernetzungsgrad etc.) negativ beeinflussen.

Auf der Suche nach einer wirtschaftlicheren, für eine heterogen katalysierte Partialoxidation zu Acrylsäure verwendbaren, Propylenquelle, wurde auch schon vorgeschlagen, von Roh-Propan auszugehen und dieses in einer der Propylenpartialoxidation vorgeschalteten Reaktionsstufe durch homogene und/oder heterogen katalysierte Oxydehydrierung und/oder heterogen katalysierte Dehydrierung partiell in Propylen umzusetzen und letzteres ohne es von nicht umgesetztem Propan aufwendig abzutrennen für die relevante Partialoxidation einzusetzen (vgl. z. B. WO 03/076370, WO 01/96271, EP-A 117 146, WO 03/011804, US-A 3,161,670, DE-A 33 13 573, WO 01/96270 und den in diesen Schriften referierten Stand der Technik). Gemäß der DE-A 102 46 119 und der DE-A 102 45 585 soll dabei so vorgegangen werden, dass durch geeignete Trennschritte dafür gesorgt wird, dass das resultierende Reaktionsgasausgangsgemisch für die Propylenpartialoxidation möglichst keine C₄-Kohlenwasserstoffe als unerwünschte, die Katalysatorperformance beeinträchtigende, Verunreinigungen enthält. Nachteilig an einer solchen Verfahrensweise ist, dass die vorgenannten Trennoperationen aufwendig, und für ein lediglich als Acrylsäurerohstoff anvisiertes Roh-Propylen wirtschaftlich gegebenenfalls prohibitiv sind, bzw. bei wirtschaftlicher Anwendung nur eine limitierte Trennwirkung erzielen.

Gleichzeitig sind Roh-Propane, die in nennenswertem Umfang gesättigte oder ungesättigte C₄-Kohlenwasserstoffe enthalten, am Markt besonders preisgünstig verfügbar, fallen sie entweder auf dem Weg zur Herstellung von Reinst-Roh-Propan als schlecht verwertbare Nebenströme an, oder wurde im Rahmen ihrer Erzeugung auf eine aufwendige C₃-/C₄-Kohlenwasserstofftrennung völlig verzichtet.

Ein solchermaßen günstiger Rohstoffpreis vermag nun im Einzelfall eine bei einer nachfolgenden heterogen katalysierten partialoxidativen Acrylsäureherstellung einhergehende Minderung der Katalysatorperformance bzw. einen frühzeitig erforderlichen Katalysatorwechsel wirtschaftlich überzukompensieren.

Verbleibender Nachteil einer ansonsten wie beschrieben gegebenenfalls attraktiven Verfahrensweise zur Herstellung von Acrylsäure ist, dass mit ihr je nach für die heterogen katalysierte Partialoxidation von mit entsprechenden C₄-Kohlenwasserstoffgehalten behaftetem Propylen zur Acrylsäureherstellung verwendeten Katalysatoren eine Methacroleinnebenkomponentenbildung infolge einer zur Propylenhauptpartialoxidation parallel erfolgenden Partialoxidation der C₄-Kohlenwasserstoffe (z. B. iso-Buten und iso-Butan) mit den beschriebenen Nachteilen einhergeht (vgl. DE-A 102 19 686, DE-A 33 13 573 und EP-A 297 445). Die selben Nachteile können je nach verwendetem Katalysator und Reaktionsbedingungen erwachsen, wenn Acrylsäure durch partielle Direktoxidation von C₄-Kohlenwasserstoffe als Verunreinigungen aufweisendem Propan erzeugt wird, wie es z. B. in der EP-A 608 838, der DE-A 198 35 247 und auch in den Schriften DE-A 102 45 585 und der DE-A 102 46 119 ausgeführt ist. Ein anderer möglicher mit C₄-Kohlenwasserstoffen oder deren oxidativen Abkömmlingen gegebenenfalls belasteter C₃-Voläufer für eine heterogen katalysierte partialoxidative Herstellung von Acrylsäure ist Acrolein (vgl. EP-A 700 893 und EP-A 700 714).

Die Aufgabe der vorliegenden Erfindung bestand daher darin, ein möglichst effizientes Verfahren zur Abtrennung von Methacrolein aus Acrylsäure als Hauptbestandteil sowie Zielprodukt, und Methacrolein als Nebenkomponente enthaltender flüssiger Phase zur Verfügung zu stellen.

Demgemäß wurde ein Verfahren zur Abtrennung von Methacrolein aus Acrylsäure als Hauptbestandteil sowie Zielprodukt, und Methacrolein als Nebenkomponente enthaltender flüssiger Phase P gefunden, das dadurch gekennzeichnet ist, dass die flüssige Phase P, bezogen auf die in ihr enthaltene Acrylsäuremenge, 0,01 bis 15 Gew.-% Methacrolein enthält und die Abtrennung kristallisativ erfolgt, wobei sich die Acrylsäure im gebildeten Kristallisat und das Methacrolein in der verbleibenden Mutterlauge anreichert.

Eine wie vorstehend beschriebene Verfahrensweise ist nur dann effizient, wenn bei der Ausbildung eines Acrylsäurekristalls im wesentlichen kein Einbau des Methacrolein in den Kristall erfolgt. Dies ist in der Regel dann der Fall, wenn der mit der Kristallisation einhergehende Abreicherungskoeffizient A^{MAC} ≥15 beträgt. Unter dem Abreicherungskoeffizienten A wird dabei generell das Mengenverhältnis von in der Mutterlauge verbliebener Verunreinigung zu im Kristallisat verbliebener Verunreinigung verstanden (jeweils ausgedrückt als Gew.-% bezogen auf die Gesamtmenge an Mutterlauge bzw. die Gesamtmenge an Kristallisat; z. B. durch Zentrifugieren oder durch Zentrifugieren und/oder Waschen können Mutterlauge und Kristallisat im wesentlichen vollständig voneinander getrennt und durch nachfolgende Analyse A bestimmt werden; eine Mutterlaugenabtrennung zu zu mehr als 90 Gew.-%, vorzugsweise zu zu mehr als 95, oder 97 oder 98, oder 99 Gew.-% ihrer Gesamtmenge ist dazu in der Regel ausreichend).

Im Fall von Essigsäure (A^{ES}) und Propionsäure (A^{PS}) als Acrylsäureverunreinigungen liegt der Abreicherungskoeffizient üblicherweise bei Werten ≤10. D. h., sie werden in die Acrylsäurekristalle mit eingebaut und sind aus diesen Kristallen z. B. durch geeignetes Waschen derselben nur schwer zu extrahieren. D. h., eine kristallisative Abtrennung dieser beiden Verunreinigungen von Acrylsäure erfordert in der Regel die Anwendung wenig effizienter und investitionsintensiver mehrstufiger Kristallisationsverfahren, wie sie z. B. in der EP-A 616 998 in Form einer mehrstufigen Kombination von dynamischer und statischer Kristallisation empfohlen wird und die neben der Mehrstufigkeit wenigstens eines dynamischen und wenigstens eines statischen Kristallisators bedarf. Allenfalls unter besonderen Rahmenbedingungen der Kristallisation (vgl. insbesondere WO 03/078378 und WO 01/77056) bilden sich Acrylsäurekristallformen aus, aus denen durch nachfolgendes Waschen mit reiner Acrylsäureschmelze Essigsäure und Propionsäure vergleichsweise gut entfernt werden können.

Je größer A^{MAC} ist, desto attraktiver ist eine kristallisative Abtrennung des Methacrolein von Acrylsäure.

In überraschender Weise wurde als Ergebnis eingehender Untersuchungen gefunden, dass bei der kristallisativen Abtrennung von Methacrolein aus Acrylsäure als Hauptbestandteil sowie Zielprodukt und Methacrolein als Nebenkomponente enthaltender flüssiger Phase selbst bei alleiniger zentrifugaler Mutterlauge/Kristallisat-Abtrennung in der Regel Abreicherungskoeffizienten A^{MAC} von bis zu 30 (in Worten: dreißig) und mehr einhergehen (dem entsprechen bei einer Waschkolonnenabtrennung Werte für A^{MAC} von wenigstens ≥ 100, in günstigen Fällen von ≥ 1000). Dieser Befund war aufgrund der chemischen Verwandtschaft beider Verbindungen (beide sind ausgeprägte Wasserstoffbrückenbildner) nicht zu erwarten. Da ihr Siedeverhalten bei Normaldruck (Sdp. Acrylsäure = 141°C, Sdp. Methacrolein = 69°C) voneinander ausgeprägt verschieden ist, wird eine z. B. rektifikative Abtrennung von Methacrolein nahegelegt. Der vorstehende physikalische Sachverhalt belegt auch, dass bei den im Stand der Technik in der Regel durchgeführten kristallisativen Reinigungen von Acrylsäure, die mittels anderer thermischer Trennverfahren bereits vorgereinigt wurde (vgl. z. B. EP-A 616 998), Acrylsäuren weitergereinigt werden, die völlig frei von Methacrolein sind. Frei von Methacrolein meint dabei in dieser Schrift, dass Methacrolein gaschromatographisch nicht mehr nachweisbar ist.

Die erfindungsgemäße Verfahrensweise eröffnet mit den vorgenannten experimentellen Befunden die Möglichkeit, auf dem Weg zur Herstellung von superabsorbertauglicher Reinacrylsäure die einer solchen Verwendung im Weg stehenden Methacroleinverunreinigungen in einem einzigen Trennschritt, in einer einzigen Kristallisationsstufe in befriedigender Weise abzutrennen.

Der Wortlaut "Acrylsäure als Hauptbestandteil sowie Zielprodukt und Methacrolein als Nebenkomponente enthaltende flüssige Phase P (bzw. Gasgemisch oder Produktgasgemisch)" soll in dieser Schrift lediglich bedeuten, dass die flüssige Phase P (bzw. das Gasgemisch oder Produktgasgemisch) Acrylsäure und Methacrolein in einem molaren Mengen-Verhältnis V von Acrylsäure zu Methacrolein von wenigstens 3 zu 2 aufweist. Selbstverständlich kann V beim erfindungsgemäßen Verfahren aber auch wenigstens 2 : 1, oder wenigstens 3 : 1, oder wenigstens 4 : 1, oder wenigstens 5 : 1, oder wenigstens 6 : 1, oder wenigstens 7 : 1, oder wenigstens 8 : 1, oder wenigstens 9 : 1, oder wenigstens 10 : 1 betragen. Das erfindungsgemäße Verfahren ist aber auch dann zweckmäßig, wenn V wenigstens 15 : 1, oder wenigstens 20 : 1, oder wenigstens 25 : 1, oder wenigstens 30 : 1, oder wenigstens 35 : 1, oder wenigstens 40 : 1, oder wenigstens 45 : 1, oder wenigstens 50 : 1, oder wenigstens 60 : 1, oder wenigstens 70 : 1, oder wenigstens 80 : 1, oder wenigstens 90 : 1, oder wenigstens 100 : 1 beträgt.

In vielen anwendungstechnisch relevanten Fällen wird V wenigstens 200 : 1, oder wenigstens 300 : 1, oder wenigstens 400 : 1, oder wenigstens 500 : 1, oder wenigstens 600 : 1, oder wenigstens 700 : 1, oder wenigstens 800 : 1, oder wenigstens 900 : 1, oder wenigstens 1000 : 1 betragen.

Das erfindungsgemäße Verfahren ist aber selbst dann noch bedeutsam, wenn V wenigstens 2000 : 1, oder wenigstens 3000 : 1, oder wenigstens 4000 : 1, oder wenigstens 5000 : 1, oder wenigstens 6000 : 1, oder wenigstens 7000 : 1, oder wenigstens 8000 : 1, oder wenigstens 9000 : 1, oder wenigstens 10000 : 1 beträgt. Selbstredend kann V beim erfindungsgemäßen Verfahren aber auch wenigstens 20000 : 1, oder wenigstens 30000 : 1, oder wenigstens 40000 : 1, oder wenigstens 50000 : 1, oder wenigstens 60000 : 1, oder wenigstens 70000 : 1, oder wenigstens 80000 : 1, oder wenigstens 90000 : 1, oder wenigstens 100000 : 1 betragen.
D. h., das erfindungsgemäße Verfahren ist insbesondere im Rahmen einer großtechnischen Herstellung von Acrylsäure noch dann von Bedeutung, wenn die flüssige Phase P (bzw. das Gasgemisch oder Produktgasgemisch), bezogen auf deren Gehalt an Acrylsäure, nur 10 Gew.-ppm an Methacrolein enthält.
D. h., V kann beim erfindungsgemäßen Verfahren z. B. 3 : 2 bis 100000 : 1, oder 2 : 1 bis 70000 : 1, oder 3 : 1 bis 50000 : 1, oder 4 : 1 bis 30000 : 1, oder 5 : 1 bis 10000 : 1, oder 6 : 1 bis 8000 : 1, oder 7 : 1 bis 6000 : 1, oder 8 : 1 bis 5000 : 1, oder 9 : 1 bis 2000 : 1, oder 10 : 1 bis 1000 : 1, oder 20 : 1 bis 800 : 1, oder 30 : 1 bis 600 : 1, oder 40 : 1 bis 400 : 1, oder 50 : 1 bis 300 : 1, oder 60 : 1 bis 200 : 1, oder 70 : 1 bis 100 : 1 betragen.

Die vorgenannten Ausführungen sind insbesondere dann zutreffend, wenn die flüssige Phase P wenigstens 10 Gew.-% Acrylsäure, oder wenigstens 20 Gew.-% Acrylsäure, oder wenigstens 30 Gew.-% Acrylsäure, oder wenigstens 40 Gew.-% Acrylsäure, oder wenigstens 50 Gew.-% Acrylsäure, oder wenigstens 60 (bzw. wenigstens 65 Gew.-%) Gew.-% Acrylsäure, oder wenigstens 70 Gew.-% Acrylsäure, oder wenigstens 80 Gew.-% Acrylsäure, oder wenigstens 90 Gew.-% Acrylsäure, oder wenigstens 93 Gew.-% Acrylsäure, oder wenigstens 94 Gew.-% Acrylsäure, oder wenigstens 95 Gew.-% Acrylsäure, oder wenigstens 96 Gew.-% Acrylsäure, oder wenigstens 97 Gew.-% Acrylsäure, oder wenigstens 98 Gew.-% Acrylsäure, oder wenigstens 99 Gew.-% Acrylsäure, oder wenigstens 99,5 Gew.-% Acrylsäure, oder wenigstens 99,7 Gew.-% Acrylsäure, oder wenigstens 99,9 Gew.-% Acrylsäure, oder wenigstens 99,95 Gew.-% Acrylsäure, oder noch mehr Acrylsäure enthält.

Bezogen auf die in den vorgenannten flüssigen Phasen P enthaltenen Acrylsäuremengen, kann deren Methacroleingehalt in für das erfindungsgemäße Verfahren typischer Weise 0,001 bis 15 Gew.-%, oder 0,01 bis 15 Gew.-%, oder 0,02 Gew.-% bis 10 Gew.-%, oder 0,03 Gew.-% bis 7 Gew.-%, oder 0,04 Gew.-% bis 5 Gew.-%, oder 0,05 Gew.-% bis 3 Gew.-%, oder 0,07 Gew.-% bis 2 Gew.-%, oder 0,09 Gew.-% bis 1,5 Gew.-%, oder 0,1 bzw. 0,15 bis 1 oder bis 0,5 Gew.-% betragen.

Ob sich beim Abkühlen von solchermaßen zusammengesetzten flüssigen Phasen P Acrylsäurekristalle abscheiden oder nicht, hängt im Einzelfall von der Gesamtzusammensetzung der flüssigen Phase P ab. Gemäß der Lehre der WO 03/078378 kann dies selbst noch bei flüssigen Phasen P der Fall sein, die 0,5 bis 90 Gew.-%, oder 7 bis 50 Gew.-%, oder 10 bis 25 Gew.-%, oder auch 10 bis 85 Gew.-%, bzw. 15 bis 80 Gew.-%, oder 25 bis 75 Gew.-% Wasser enthalten.

Aus der EP-A 002 612 ist bekannt, dass das Eutektikum von Wasser-Acrylsäure z. B. durch Zusatz von Salzen zu wässrigen Acrylsäurelösungen aufgebrochen und so auch bei niederen Acrylsäuregehalten eine Kristallisation der Acrylsäure bewirkt werden kann (diese Hilfsmaßnahme kann auch beim erfindungsgemäßen Verfahren angewendet werden).

Die WO 99/06348 empfiehlt in ähnlicher Weise den Zusatz von polaren organischen Substanzen vorab einer kristallisativen Acrylssäureabtrennung aus wässrigen Phasen P (diese Hilfsmaßnahme kann auch beim erfindungsgemäßen Verfahren angewendet werden).

Die DE-A 198 38 845 lehrt, dass Acrylsäure aus flüssigen Phasen P, die Acrylsäure und ein unter Normalbedingungen höher als Acrylsäure siedendes organisches Lösungsmittel aufweisen, beim Abkühlen in der Regel dann abgeschieden wird, wenn die flüssige Phase P > 60 bis < 99,9 Gew.-% Acrylsäure, 0,1 bis 40 Gew.-% hochsiedendes organisches Lösungsmittel und > 0 bis 35 Gew.-% bei der gasphasenkatalytischen Acrylsäureherstellung anfallende Nebenkomponenten enthält. Die flüssigen Phasen P der DE-A 198 38 845, die einen integralen Bestandteil dieser Schrift bildet, kommen auch für das erfindungsgemäße Verfahren in Betracht, sofern sie die erfindungsgemäß erforderliche Methacroleinverunreinigung aufweisen.

Gemäß dem vorstehenden ist davon auszugehen, dass wenigstens beim Abkühlen von ≥ 65 Gew.-% Acrylsäure enthaltenden, erfindungsgemäß zu behandelnden flüssigen Phasen P regelmäßig Acrylsäure auskristallisiert.

Das erfindungsgemäße Verfahren ist daher vor allem auf mit Methacrolein verunreinigte flüssige Phasen P vorteilhaft anwendbar, wenn diese 65 bis 99,5 Gew.-%, oder 70 bis 99,5 Gew.-%, oder 80 bis 99,5 Gew.-%, oder 85 bis 99 Gew.-%, oder 90 bis 98 Gew.-%, oder 93 bis 97 Gew.-% Acrylsäure enthalten. Dies vor allem dann, wenn die Methacroleingehalte, bezogen auf enthaltene Acrylsäure gleichzeitig 0,01 bis 15 Gew.-%, oder 0,02 bis 10 Gew.-%, oder 0,03 bis 7 Gew.-%, oder 0,04 bis 5 Gew.-%, oder 0,05 bis 3 Gew.-%, oder 0,07 bis 2 Gew.-%, oder 0,09 bis 1,5 Gew.-%, oder 0,1 bzw. 0,15 bis 1 oder bis 0,5 Gew.-% betragen.

Im übrigen können erfindungsgemäß zu behandelnde flüssige Phasen P in an sich bekannter Weise aus Acrylsäure als Hauptbestandteil und Methacrolein als Nebenkomponente enthaltenden Produktgasgemischen von heterogen katalysierten Partialoxidationen von z.B. in beschriebener Weise verunreinigten C₃-Vorläufern der Acrylsäure (Propan, Propylen und/oder Acrolein; als C₃-Vorläufer kommen auch Propionsäure, Propanol und/oder Propionaldehyd in Betracht; in diesem Fall ist die Partialoxidation eine heterogen katalysierte Oxidehydrierung) gewonnen werden (auf diese zurückgehen), wie dies im Stand der Technik bereits beschrieben ist. Dabei wird die sonstige Zusammensetzung dieser Produktgasgemische im wesentlichen derjenigen entsprechen, wie es aus den bekannten gasphasenoxidativen Acrylsäureherstellungen bekannt ist. Ferner kann das erfindungsgemäße Kristallisationsverfahren auf gleiche Weise ausgeübt und auf gleiche Weise in das Gesamtverfahren zur Abtrennung von (Rein) Acrylsäure aus dem Produktgemisch integriert sein, wie es insbesondere die folgenden Schriften des Standes der Technik lehren, die allesamt integraler Bestandteil dieser Schrift sind (typische Methacroleingehalte von solchen Produktgasgemischen liegen, bezogen auf die darin enthaltene Menge an Acrylsäure, z. B. bei 0,01 bis 15, oder bis 10, oder bis 5 Gew.-%, häufig bei 0,02 bis 4 Gew.-%, vielfach bei 0,03 bis 3 Gew.-%, oft bei 0,04 bis 2 Gew.-%, aber auch bei 0,05 bis 1 Gew.-%, oder bei 0,07 bis 0,75 Gew.-%, bzw. bei 0,1 bis 0,5 Gew.-%, oder bei 0,2 bis 0,4 Gew.-%): die WO 02/055469, die WO 03/078378, die WO 01/77056, die WO 03/041833, die DE-A 196 06 877, die DE-A 103 36 386, die WO 98/01414, die WO 01/77056, die EP-A 14 84 308, die EP-A 14 84 309, die US-A 2004/0242826, die DE-A 102 43 625, die DE-A 196 06 877, die EP-A 792 867, die EP-A 10 15 410, die EP-A 920 408, die EP-A 11 89 861, die EP-A 10 15 411, die EP-A 10 68 174, die WO 2004/035514, die EP-A 10 66 293, die EP-A 11 63 201, die EP-A 1159 249, die WO 02/090310, die DE-A 101 22 787, die WO 03/041832, die DE-A 102 35 847, die EP-A 12 52 129, die EP-A 616 998, die EP-A 13 88 533, die EP-A 11 25 912 und die EP-A 11 16 709.

Von ganz besonderer Bedeutung ist das erfindungsgemäße Verfahren dann, wenn man die erfindungsgemäß zu behandelnde, Acrylsäure als Hauptbestandteil sowie Zielprodukt und Methacrolein als Nebenkomponente enthaltende flüssige Phase P aus dem Produktgasgemisch einer heterogen katalysierten partiellen Gasphasenoxidation wenigstens eines C₃-Vorläufers der Acrylsäure unter Anwendung wenigstens eines unscharfen Trennverfahrens erzeugt. Dies insbesondere dann, wenn man bei der nachfolgenden kristallisativen Abtrennung des verbliebenen Methacrolein verbliebene Mutterlauge wenigstens teilweise in wenigstens eines der unscharfen Trennverfahren rückführt.

Die Grundstruktur einer solchen kombinierten Anwendung von unscharfen Trennverfahren und dem scharfen Trennverfahren der Kristallisation, lehren z. B. die DE-A 196 06 877, die EP-A 792 867 sowie die EP-A 14 84 308, die EP-A 14 84 309, die EP-A 11 16 709 und insbesondere die EP-A 10 15 410.

Ein unscharfes Trennverfahren ist dabei als ein Trennverfahren definiert, bei dem die Zusammensetzung der sich bei Anwendung des Trennverfahrens bildenden, Zielprodukt angereichert enthaltenden, Phase von der Zusammensetzung des zu trennenden Gemischs in ausgeprägter Weise abhängig ist, während die erfindungsgemäße kristallisative Behandlung insofern ein scharfes Trennverfahren ist, da die Zusammensetzung der sich bildenden Acrylsäurekristalle weitgehend unabhängig (im Idealfall besteht völlige Unabhängigkeit) von der Zusammensetzung der flüssigen Phase P ist.

Das erfindungsgemäße Verfahren ist im Fall einer solchen Kombination eines scharfen und eines unscharfen Trennverfahrens insofern von erhöhter Bedeutung, als sich beim kontinuierlichen Betrieb einer solchen Verfahrensweise das Methacrolein in der erfindungsgemäß zu behandelnden flüssige Phase P durch die Mutterlaugerückführung aufpegelt, da die Mutterlauge das Methacrolein angereichert enthält. D. h., auch vergleichsweise geringe Methacroleingehalte im Produktgasgemisch der Gasphasenoxidation können sich so zu einem ernsten Problem auswachsen. Erhöhte Methacroleingehalte in flüssigen Phasen P können auch z. B. dann enthalten sein, wenn beim erfindungsgemäßen Verfahren anfallende Mutterlaugen zum Zweck der Ausbeutesteigerung weiterkristallisiert werden, oder wenn in den unscharfen Trennverfahren anfallende, Methacrolein enthaltende Nebenstoffströme zur Ausbeutesteigerung erfindungsgemäß behandelt werden.

Dies bedeutet, dass im Fall von Abreicherungskoeffizienten A^{MAC} < 15 eine solche Verfahrensweise äußerst ineffizient wäre. Die großtechnisch erforderliche Effizienz erlangt sie nur dadurch, dass erfindungsgemäß überraschend ein A^{MAC} von > 15 gefunden wurde.

Im Regelfall wird das dabei wenigstens eine zur Erzeugung der erfindungsgemäß zu behandelnden flüssigen Phase P aus dem Produktgasgemisch einer heterogen katalysierten partiellen Gasphasenoxidation wenigstens eines C₃-Vorläufers der Acrylsäure angewandte unscharfe Trennverfahren eine Destillation, Rektifikation, Absorption, Adsorption, Extraktion, Desorption, Strippung, Destraktion, (partielle) Kondensation, fraktionierende Kondensation, ein Membrantrennverfahren wie eine Pervaporation/Dämpferpermeation oder eine Kombination solcher Verfahren sein.

Besonders häufig wird man eine Destillation, Rektifikation, Absorption, Extraktion, partielle Kondensation, fraktionierende Kondensation, Desorption, Strippung und/oder Destraktion anwenden. Häufig wird man zur Erzeugung der erfindungsgemäß zu behandelnden flüssigen Phase P vorgenannte Verfahren mehrfach anwenden.

Im einfachsten Fall kann die erfindungsgemäß zu behandelnde flüssige Phase P das Absorbat und/oder partielle und/oder fraktionierend erhaltene Kondensat einer absorptiven und/oder kondensativen Abtrennung der Acrylsäure aus dem Produktgasgemisch einer heterogen katalysierten partiellen Gasphasenoxidation wenigstens eines der in dieser Schrift aufgeführten C₃-Vorläufer sein. Erfindungsgemäß bevorzugt erfolgt dann Mutterlaugerückführung in die Absorption und/oder Kondensation.

In zweckmäßiger Weise weist eine wie beschrieben anzuwendende Kombination aus unscharfer und scharfer Trennung einen Auslass für wenigstens einen Methacrolein angereichert enthaltenden Stoffstrom auf. Erfindungsgemäß vorteilhaft liegt dieser auf der Seite der unscharfen Trennverfahren. In der Regel wird man als solchen Auslass die Sumpfflüssigkeit einer Trennkolonne verwenden, aus der die flüssige Phase P selbst oder der im weiteren Verlauf in die flüssige Phase P zu wandelnde Stoffstrom z.B. über Seitenentnahme und/oder über Kopfentnahme entnommen wird.

Ein Methacroleinauslass kann sich aber auch oder nur auf der Seite der erfindungsgemäßen Abtrennung, d.h., auf der kristallisativen Seite befinden. In diesem Fall wird der Auslass aus Methacrolein angereichert enthaltender Mutterlauge bestehen.

Wird die erfindungsgemäße Abtrennung z.B. mittels einer Kombination von dynamischer und statischer Kristallisation gemäß der EP-A 616998 ausgeführt, wird sich der Methacrolein angereichert enthaltende Mutterlaugeauslass im Bereich der statischen Kristallisation befinden. Letzteres insbesondere dann, wenn bei Anwendung des erfindungsgemäßen Verfahrens keine Mutterlaugerückführung in eine unscharfe Trennung durchgeführt wird.

Das erfindungsgemäße Verfahren ist nicht zuletzt dann günstig, wenn die erfindungsgemäß zu behandelnde flüssige Phase P (z. B. über eine der vorstehend beschriebenen Verfahrensweisen) auf ein Produktgasgemisch einer Partialoxidation wenigstens eines C₃-Vorläufers der Acrylsäure zurückgeht, das das Methacrolein in den bereits genannten, auf den Acrylsäuregehalt des Produktgasgemischs bezogenen, Gehalten aufweist und im übrigen enthält:
1 bis 30 Vol.-% Acrylsäure,
≥ 0 bzw. 0,005 bis 10 Vol.-% Propylen,
≥ 0 bzw. 0,001 bis 2 Vol.-% Acrolein,
≥ 0 bzw. 0,001 bis 2 Vol.-% Methacrylsäure,
≥ 0 bzw. 0,005 bis 10 Vol.-% molekularer Sauerstoff,
≥ 0 bzw. 0,005 bis 3 Vol.-% Essigsäure,
≥ 0 bzw. 0,001 bis 2 Vol.-% Propionsäure,
≥ 0 bzw. 0,001 bis 2 Vol.-% Formaldehyd,
≥ 0 bzw. 0,001 bis 2 Vol.-% sonstige von Methacrolein verschiedene Aldehyde,
und 10 bis 98 bzw. 50 bis 98 Vol.-% (inerte) Verdünnungsgase.

Die Verdünnungsgase können enthalten:
≥ 0 bzw. 0,005 bis 90 Vol.-% gesättigte C₁- bis C₆-Kohlenwasserstoffe (insbesondere Propan, Methan und/oder Ethan),
≥ 0 bzw. 0,05 bis 30 Vol.-% Wasserdampf,
≥ 0 bzw. 0,05 bis 15 Vol.-% Kohlenoxide (CO und/oder CO₂),
und ≥ 0 bzw. 1 bis 90 Vol.-% molekularer Stickstoff.

Dabei kann das Partialoxidationsproduktgasgemisch insbesondere auf eine Partialoxidation zurückgehen, wie sie in den Schriften DE-A 10 2004 032 129 und deren äquivalenten Auslandsschutzrechten, DE-A 10245585, WO 03/076370, WO 01/96271, EP-A 117146, WO 03/011804, US-A 3161670, DE-A 3313573, DE-A 10316039 und WO 01/96270 ausgehend von Propylen und/oder Propan beschrieben ist, und als Propylenquelle gegebenenfalls eine Propandehydrierung und/oder -oxidehydrierung (gegebenenfalls heterogen katalysiert) als vorgeschaltete Reaktionsstufe aufweist.

Als repräsentative (d.h., eine beispielhaft mögliche) solche Produktgasgemischzusammensetzung sei beispielhaft ein Produktgasgemisch genannt, das enthält (alle aufgeführten Nebenkomponenten würden bei Anwendung des erfindungsgemäßen Verfahrens in zufriedenstellender Weise mitabgetrennt, falls sie Bestandteil der flüssigen Phase P wären):

| | Vol.-% |
|---|---|
| Stickstoff | 51,54 |
| Sauerstoff | 2,3 |
| Propan | 29,20 |
| Propen | 0,110 |
| Methan | 0 |
| Ethan | 0,077 |
| n-Butan | 0,101 |
| iso-Butan | 0,236 |
| n-Butene | 0 |
| iso-Buten | 0,001 |
| 1,3-Butadien | 0,009 |
| Wasserstoff | 0,05 |
| Kohlenmonoxid | 0,596 |
| Kohlendioxid | 1,72 |
| Wasser | 8,21 |
| Acrolein | 0,09 |
| Acrylsäure | 5,28 |
| Essigsäure | 0,240 |
| Propionsäure | 0,002 |
| Ameisensäure | 0,019 |
| Formaldehyd | 0,198 |
| Benzaldehyd | 0,005 |
| Maleinsäureanhydrid | 0,047 |
| Methacrolein | 0,020 |
| Methacrylsäure | 0,011 und |
| Ethen | 0,032. |

Erfindungsgemäß vorteilhaft wird man die erfindungsgemäß zu behandelnde flüssige Phase P aus den vorgenannten Produktgasgemischen der C₃-Acrylsäurevorläuferpartialoxidation dadurch erzeugen, dass man Acrylsäure aus dem Produktgasgemisch auskondensiert. Das dabei anfallende Kondensat bildet erfindungsgemäß vorteilhaft unmittelbar die flüssige Phase P. Mit Vorteil erfolgt das Auskondensieren der Acrylsäure aus dem (zuvor gegebenenfalls abgekühlten) Produktgasgemisch als fraktionierende Kondensation (der gegebenenfalls zusätzlich eine Absorption mit Wasser und/oder wässrige Lösung überlagert wird, um Acrylsäureverluste zu mindern), wie es z.B. in den Schriften EP-A 1015410, WO 2004/035514, DE-A 10243625, EP-A 1015411, DE-A 10235847, EP-A 1159249, EP-A 1163201, EP-A 1066239 und EP-A 920408 ausführlich beschrieben ist.

Dabei wird das Produktgasgemisch zweckmäßig, gegebenenfalls nach zuvor erfolgter direkter und/oder indirekter (z.B. mit einer Quenchflüssigkeit gemäß der EP-A 1066239, oder gemäß der EP-A 1163201) Kühlung in einer trennwirksame Einbauten aufweisenden Trennkolonne in sich selbst aufsteigend unter Seitenabzug einer rohen Acrylsäure (die vorzugsweise die erfindungsgemäß zu behandelnde flüssige Phase P bildet; gegebenenfalls wird die rohe Acrylsäure zur Erzeugung der flüssigen Phase P noch rektifikativ und/oder destillativ behandelt) fraktionierend kondensiert.

Solchermaßen kondensativ (und gegebenenfalls zusätzlich rektifikativ) erzeugte flüssige Phase P kann nun erfindungsgemäß kristallisativ behandelt werden. Dabei gebildete, Methacrolein angereichert enthaltende Mutterlauge wird man gemäß dem Vorbild z.B. der EP-A 920408 bzw. WO 2004/035514 wenigstens teilweise, vorzugsweise vollständig, in die Kondensation der Acrylsäure aus dem Produktgasgemisch rückführen. Den Methacroleinauslaß wird man dabei unterhalb des Seitenabzugs der rohen Acrylsäure ansiedeln.

Solchermaßen durch Partial- oder Totalkondensation und/oder überlagerte Absorption mit Wasser oder wässriger Lösung sowie gegebenenfalls rektifikative Nachbehandlung erzeugte, erfindungsgemäß zu behandelnde flüssige Phase P, kann neben den bereits aufgezeigten (erfindungsgemäß wesentlichen), auf enthaltene Acrylsäure bezogenen Gehalten an Methacrolein, enthalten:
65, oder 75, oder 85 bis 99,5 Gew.-% Acrylsäure,
≥ 0, in der Regel 0,1 bis 40 Gew.-% Wasser,
≥ 0, in der Regel 0,001 bis 5 Gew.-% Acrolein,
≥ 0, in der Regel 0,001 bis 10 Gew.-% Methacrylsäure,
≥ 0, in der Regel 0,01 bis 5 Gew.-% Essigsäure,
≥ 0, in der Regel 0,01 bis 5 Gew.-% Propionsäure,
≥ 0, in der Regel 0,001 bis 5 Gew.-% Formaldehyd,
≥ 0, in der Regel 0,001 bis 5 Gew.-% weitere von Methacrolein verschiedene Aldehyde (je Aldehyd), und
≥ 0, in der Regel 0,01 bis 5 Gew.-% Maleinsäure.

Mit Vorteil werden die vorgenannten Gehalte der flüssigen Phase P betragen:
93 bis 98 Gew.-% Acrylsäure,
1 bis 5 Gew.-% Wasser,
0,001 bis 3 Gew.-% Acrolein,
0,001 bis 3 Gew.-% Methacrylsäure,
0,1 bis 3 Gew.-% Essigsäure,
0,01 bis 3 Gew.-% Propionsäure,
0,001 bis 3 Gew.-% Formaldehyd,
0,001 bis 3 Gew.-% weitere Aldehyde (je Aldehyd) und
0,01 bis 3 Gew.-% Maleinsäure.

Auch kann sie den Angaben der WO 02/055469 bzw. WO 03/078378 folgend bis zu 3 Gew.-% Protoanemonin enthalten.

Die erfindungsgemäße kristallisative Behandlung der flüssigen Phase P, insbesondere einer in vorgenannter Weise kondensativ und/oder absorptiv und/oder rektifikativ gewonnenen flüssigen Phase P, unterliegt grundsätzlich keiner Beschränkung, einschließlich den Verfahren zur Abtrennung der Mutterlauge vom Kristallisat (alle im genannten Stand der Technik ausgeführten Verfahren sind anwendbar).

D.h., sie kann einstufig, oder mehrstufig, kontinuierlich oder diskontinuierlich durchgeführt werden. Insbesondere kann sie auch als fraktionierte Kristallisation durchgeführt werden. Üblicherweise werden bei einer fraktionierten Kristallisation alle Stufen, die ein Acrylsäurekristallisat erzeugen, das (insbesondere an Methacrolein) reiner als die zugeführte flüssige Phase P ist, Reinigungsstufen genannt und alle anderen Stufen Abtriebsstufen. Zweckmäßig werden nun mehrstufige Verfahren nach dem Gegenstromprinzip betrieben, bei dem nach der Kristallisation in jeder Stufe das Kristallisat von der Mutterlauge abgetrennt und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

In der Regel liegt die Temperatur der flüssigen Phase P während des erfindungsgemäßen Verfahrens zwischen -25°C und +14°C, insbesondere zwischen 12°C und -5°C.

Beispielsweise kann das erfindungsgemäße Verfahren als Schichtkristallisation ausgeführt sein (vgl. DE-OS 2606364, EP-A 616998, EP-A 648520 und EP-A 776875). Dabei wird das Kristallisat in Form zusammenhängender, fest anhaftender Schichten ausgefroren. Die Trennung des abgeschiedenen Kristallisats von der verbliebenen Restschmelze (die Mutterlauge) erfolgt durch einfaches Abfließen der Restschmelze. Prinzipiell unterscheidet man zwischen "statischen" und "dynamischen" Schichtkristallisationsverfahren. Kennzeichnend für die dynamische Schichtkristallisation von flüssigen Phasen P ist eine erzwungene Konvektion der flüssigen Phase P. Diese kann durch Umpumpen der flüssigen Phase durch volldurchströmte Rohre, durch Aufgabe der flüssigen Phase P als Rieselfilm (z.B. gemäß EP-A 616998) oder durch Einleiten von Inertgas in eine flüssige Phase P oder durch Pulsieren erfolgen.

Bei den statischen Verfahren ruht die flüssige Phase P (z.B. in Rohrbündel- oder Plattenwärmetauschern) und scheidet sich schichtförmig durch langsame Temperatursenkung auf der Sekundärseite ab. Danach wird die Restschmelze (Mutterlauge) abgelassen, durch langsame Temperaturerhöhung stärker verunreinigte Fraktionen aus der Kristallschicht abgeschwitzt und nachfolgend das Reinprodukt abgeschmolzen (vgl. WO 01/77056).

Erfindungsgemäß bevorzugt wird das erfindungsgemäße Verfahren im Fall aller in dieser Schrift beschriebenen flüssigen Phasen P jedoch gemäß der Lehre der WO 01/77056, der WO 02/055469 und WO 03/078378 als Suspensionskristallisation ausgeführt.

Dabei wird in der Regel durch Kühlung der flüssigen Phase P eine Acrylsäurekristalle suspendiert enthaltende Kristallsuspension erzeugt, wobei die Acrylsäurekristalle einen geringeren und die verbleibende Restschmelze (Mutterlauge) einen höheren Methacroleingehalt (relativ bezogen auf die jeweilige Gesamtmenge) aufweist, als die zu reinigende flüssige Phase P. Dabei können die Acrylsäurekristalle unmittelbar in Suspension befindlich wachsen und/oder sich als Schicht auf einer gekühlten Wand abscheiden, von der sie anschließend abgekratzt und in der Restschmelze (Mutterlauge) resuspendiert werden.

Alle in der WO 01/77056, WO 02/055469, sowie WO 03/078378 aufgeführten Suspensionskristaller und Suspensionskristallisationsverfahren kommen erfindungsgemäß in Betracht. In der Regel weist die dabei erzeugte Acrylsäurekristallisatsuspension einen Feststoffgehalt von 20 bis 40 Gew.-% auf.

Weiterhin kommen alle in den vorgenannten WO-Veröffentlichungen genannten Verfahren zur Trennung von gebildetem Suspensionskristallisat und verbliebener Mutterlauge in Betracht (z.B. mechanische Trennverfahren wie Zentrifugieren). Erfindungsgemäß bevorzugt erfolgt die Trennung in einer Waschkolonne. Bevorzugt handelt es sich dabei um eine Waschkolonne mit erzwungenem Transport der abgeschiedenen Acrylsäurekristalle. Der Kristallvolumenanteil im Kristallbett erreicht dabei in der Regel Werte > 0,5. Im Regelfall wird die Waschkolonne bei Werten von 0,6 bis 0,75 betrieben. Als Waschflüssigkeit wird mit Vorteil die Schmelze von in der Waschkolonne vorab gereinigten (abgetrennten) Acrylsäurekristallen verwendet. Die Wäsche erfolgt normalerweise im Gegenstrom. Damit umfasst das erfindungsgemäße Verfahren insbesondere Verfahren, welche folgende Verfahrensschritte umfassen:
a) Auskristallisieren von Acrylsäure aus einer flüssigen Phase P
b) Trennen des Acrylsäurekristallisats von der verbliebenen Mutterlauge (Restschmelze, flüssigen Restphase),
c) wenigstens teilweises Aufschmelzen des abgetrennten Acrylsäurekristallisats und
d) wenigstens teilweises Rückführen des aufgeschmolzenen Acrylsäurekristallisats zum Schritt b) und/oder zum Schritt a).

Bevorzugt erfolgt dabei der Schritt b) durch Gegenstromwäsche mit in den Schritt b) rückgeführtem aufgeschmolzenem zuvor abgetrenntem Acrylsäurekristallisat.

Erfindungsgemäß vorteilhaft enthält die flüssige Phase P bei Anwendung des erfindungsgemäßen Verfahrens Wasser, da eine Ausbildung von Acrylsäurekristallisat im Beisein von Wasser gemäß der Lehre der WO 01/77056 und WO 03/078378 eine für die nachfolgende Trennung des Kristallisats von verbliebener Mutterlauge besonders günstige Kristallform bedingt. Dies gilt insbesondere dann, wenn die Kristallisation als Suspenskristallisation ausgeführt wird, und noch mehr, wenn die nachfolgende Mutterlaugeabtrennung in einer Waschkolonne ausgeführt wird, und noch mehr, wenn dabei als Waschflüssigkeit die Schmelze von in der Waschkolonne bereits gereinigtem Acrylsäurekristallisat verwendet wird.

D.h., das erfindungsgemäße Verfahren umfasst insbesondere Verfahren, bei denen man die zu reinigende flüssige Phase P unter Einwirkung von Kälte in eine aus Acrylsäurekristallisat und flüssiger Restphase (Restschmelze) bestehende Kristallisatsuspension überführt, wobei der Gewichtsanteil des Acrylsäurekristallisats an Methacrolein kleiner und der Gewichtsanteil der flüssigen Restphase (der Mutterlauge) an Methacrolein größer als der Methacroleingewichtsanteil der flüssigen Phase P ist, von der Kristallisatsuspension gegebenenfalls einen Teil der verbliebenen Mutterlauge mechanisch abtrennt und das Acrylsäurekristallisat in einer Waschkolonne von verbliebener Mutterlauge mit der Maßgabe befreit, dass
a) die flüssige Phase P bezogen auf die darin enthaltene Acrylsäure 0,20 bis 30, häufig bis 20, oft bis 10 Gew.-% an Wasser enthält, und
b) als Waschflüssigkeit die Schmelze von in der Waschkolonne gereinigtem Acrylsäurekristallisat verwendet wird.

Insbesondere umfasst das erfindungsgemäße Verfahren vorstehende Verfahren, wobei die flüssige Phase P ≥ 80 Gew.-% Acrylsäure, oder ≥ 90 Gew.-% Acrylsäure, oder ≥ 95 Gew.-% Acrylsäure aufweist. Das molare Verhältnis V in der zu reinigenden flüssigen Phase P kann dabei jeweils alle in dieser Schrift genannten Werten aufweisen.

Weiterhin ist es erfindungsgemäß vorteilhaft, wenn der Wassergehalt der flüssigen Phase P bei vorstehend beschriebenen Verfahrensweisen (bzw. ganz generell bei Anwendung des erfindungsgemäßen Verfahrens), bezogen auf in der flüssigen Phase P enthaltene Acrylsäure, 0,2 bzw. 0,4 bis 8, bzw. bis 10, bzw. bis 20, bzw. bis 30 Gew.-%, bzw. 0,6 bis 5 Gew.-%, bzw. 0,60 bis 3 Gew.-% beträgt.

Selbstredend ist das erfindungsgemäße Verfahren auch auf alle Roh-Acrylsäuren der WO 98/01414 anwendbar, soweit diese zusätzlich Methacrolein als Nebenkomponente enthalten.

Alles Vorgenannte gilt vor allem dann, wenn die Waschkolonne eine Waschkolonne mit erzwungenem Transport der Acrylsäurekristalle ist, und dies vor allem dann, wenn es eine hydraulische oder eine mechanische Waschkolonne gemäß der WO 01/77056 ist und sie wie dort ausgeführt betrieben wird.

Alles Vorgenannte gilt vor allem dann, wenn die Waschkolonne gemäß den Lehren der WO 03/041832 sowie der WO 03/041833 ausgeführt ist und betrieben wird.

Das erfindungsgemäße Verfahren gestattet so, mit der Sequenz Partialoxidation wenigstens eines C₃-Vorläufers, fraktionierende Acrylsäurekondensation aus dem Produktgasgemisch der Partialoxidation, Suspensionskristallisation des entnommenen Acrylsäurekondensats und Abtrennung des Suspensionskristallisats von verbliebener Mutterlauge in einer Waschkolonne unter Anwendung einer Reinkristallisatschmelze als Waschflüssigkeit, auf effizienteste Art und Weise und unter Anwendung lediglich einer Kristallisationsstufe die Herstellung von superabsorbertauglicher Acrylsäure (solche Acrylsäure kann selbstredend auch für alle anderen in der WO 02/055469 und WO 03/078378 angesprochenen Verwendungen eingesetzt werden und dies auch dann, wenn von einer billigen, die Nebenproduktbildung von Methacrolein bedingenden C₃-Vorläufer-Rohstoffquelle für die Partialoxidation ausgegangen wird).

Selbstverständlich werden alle in dieser Schrift aufgeführten Verfahrensschritte polymerisationsinhibiert durchgeführt. Dabei kann wie im aufgeführten Stand der Technik beschrieben vorgegangen werden. Eine herausragende Position unter der Gesamtmenge der verfügbaren Acrylsäure-Prozeßstabilisatoren nehmen Dibenzo-1,4-thiazin (PTZ), 4-Hydroxy-2,2,6,6-tetramethylpiperidin-1-oxyl (4-OH-TEMPO) und p-Methoxyphenol (MEHQ) ein, die entweder jeweils für sich, oder paarweise oder als Dreiergemisch Bestandteil der erfindungsgemäß zu behandelnden flüssigen Phase P sein können. Üblicherweise beträgt die Gesamtmenge von an in der flüssigen Phase P enthaltenen Polymerisationsinhibitoren, bezogen auf die Gesamtmenge an darin enthaltener Acrylsäure und Methacrolein 0,001 bis 2 Gew.-%.

Aufgrund unerwünschter Bildung von Acrylsäure-Oligomeren (Michael-Addukte) in der flüssigen Phase P bei deren sich selbst überlassen, wird das erfindungsgemäße Verfahren möglichst umgehend nach Erzeugung der flüssigen Phase P angewendet.

In erfindungsgemäß vorteilhafter Weise werden bei Anwendung des erfindungsgemäßen Verfahrens auch sonstige in der flüssigen Phase P enthaltene C₄- (z.B. Buten-1, Butadien, n-Butan etc.) Partialoxidationsfolgeprodukte wie z.B. Methacrylsäure, Buttersäuren, Butyraldehyde etc. mitabgetrennt. Sie können, bezogen auf Acrylsäure, in den gleichen Mengen wie Methacrolein in der flüssigen Phase P (insbesondere in allen in dieser Schrift explizit ausgeführten flüssigen Phasen P) enthalten sein. Das gleiche gilt für Propionaldehyd sowie alle C₅- und C₆-Partialoxidationsfolgeprodukte.

### Beispiel

Jeweils ca. 1800 g verschiedener Acrylsäuren werden in einen gerührten Metallkessel (2 I Innenvolumen, nahezu wandgängiger Wendelrührer) gefüllt. Sie sind durch Zusatz von 100 bis 200 Gew.ppm Monomethylether des Hydrochinons (MEHQ) und < 100 Gew.ppm Phenothiazin (bezogen auf enthaltene Acrylsäure) polymerisationsinhibiert.

Mit einer Abkühlgeschwindigkeit von 1 K/h wird die Temperatur der im Mantel geführten Kühlflüssigkeit so lange abgesenkt, bis die resultierende Kristallisatsuspension (Acrylsäuerkristalle suspendiert in Restschmelze) einen Feststoffgehalt von jeweils ca. 18 Gew.-% aufweist. Dann wird ein Teil der Kristallisatsuspension entnommen und auf einer Laborzentrifuge in einem mit einem Polypropylenfiltergewebe ausgerüsteten Siebbecher bei 2000 Umdrehungen je Minute 180 Sekunden zentrifugiert und so die verbleibende Mutterlauge nahezu vollständig abgeschleudert. Analyse des verbliebenen Kristallisats so wie der abgeschleuderten Mutterlauge ergibt für die verschieden verunreinigten Acrylsäuren (erfindungsgemäß zu behandelnde flüssige Phasen P) für die darin enthaltenen Nebenkomponenten die nachfolgend aufgelisteten Abreicherungskoeffizienten. Die Acrylsäuren werden ausgehend von einer auf eine heterogen katalysierte Gasphasenpartialoxidation von leicht C₄-haltigem Propylen zurückgehenden Acrylsäure durch Zusatz von polymerisationsinhibiertem Rein-Methacrolein zu selbiger erzeugt. Die Ausgangsacrylsäure enthält folgende Gehalte:
95,201 Gew.-% Acrylsäure,
0,042 Gew.-% Methacrolein,
0,604 Gew.-% Benzaldehyd,
0,062 Gew.-% Propionsäure,
0,687 Gew.-% Furan-2-aldehyd,
0,663 Gew.-% Essigsäure,
0,004 Gew.-% Furan-3-aldehyd,
0,002 Gew.-% Allylacrylat,
0,009 Gew.-% Acrolein und
2,20 Gew.-% Wasser.

Diese Acrylsäure wird einmal mit 0,2 Gew.-% Methacrolein (Probe 1), einmal mit 1 Gew.-% Methacrolein (Probe 2) und einmal mit 5 Gew.-% Methacrolein (Probe 3) versetzt (jeweils bezogen auf das Gewicht der Ausgangsacrylsäure) und die verschiedenen Proben wie beschrieben behandelt. In gleicher Weise wird die Ausgangssäure selbst behandelt. Für die in den verschiedenen Proben sowie in der Ausgangssäure enthaltenen Nebenkomponenten werden folgende Abreicherungskoeffizienten gefunden:

| Nebenkomponete | A (Probe 1) | A (Probe 2) | A (Probe 3) | A (Ausgangssäure) |
|---|---|---|---|---|
| Methacrolein | >15 | > 15 | >15 | im Kristallisat nicht mehr nachweisbar |
| Benzaldehyd | 31,3 | 45,4 | 31,2 | 31,2 |
| Propionsäure | 3,9 | 4,1 | 3,85 | 3,86 |
| Furan-2-aldehyd | 31,3 | 47,6 | 30,3 | 31,25 |
| Essigsäure | 5,8 | 6,4 | 5,59 | 5,85 |

Die U.S. Provisional Patent Application Nos. 60/656,882, wurde am 01.03.2005 eingereicht und 60/668,088, wurde am 05.04.2005 eingereicht.

## Patentansprüche

1. Verfahren zur Abtrennung von Methacrolein aus Acrylsäure als Hauptbestandteil sowie Zielprodukt, und Methacrolein als Nebenkomponente enthaltender flüssiger Phase P, **dadurch gekennzeichnet, dass** die flüssige Phase P, bezogen auf die in ihr enthaltene Acrylsäuremenge, 0,01 bis 15 Gew.-% Methacrolein enthält und die Abtrennung kristallisativ erfolgt, wobei sich die Acrylsäure im gebildeten Kristallisat und das Methacrolein in der verbleibenden Mutterlauge anreichert.

2. Verfahren nach Anspruch 1, **dadurch gekennzeichnet, dass** die flüssige Phase P wenigstens 10 Gew.-% Acrylsäure enthält.

3. Verfahren nach Anspruch 1 oder 2, **dadurch gekennzeichnet, dass** die flüssige Phase P wenigstens 65 Gew.-% Acrylsäure enthält.

4. Verfahren nach einem der Ansprüche 1 bis 3, **dadurch gekennzeichnet, dass** die flüssige Phase P 65 bis 99,5 Gew.-% Acrylsäure enthält.

5. Verfahren nach einem der Ansprüche 1 bis 4, **dadurch gekennzeichnet, dass** die flüssige Phase P, bezogen auf darin enthaltene Acrylsäure, 0,2 bis 30 Gew.-% Wasser enthält.

6. Verfahren nach einem der Ansprüche 1 bis 5, **dadurch gekennzeichnet, dass** die flüssige Phase P auf das Produktgasgemisch einer heterogen katalysierten Gasphasenpartialoxidation wenigstens einer C₃-Vorläuferverbindung der Acrylsäure zurückgeht.

7. Verfahren nach Anspruch 6, **dadurch gekennzeichnet, dass** die wenigstens eine C₃-Vorläuferverbindung Propan ist.

8. Verfahren nach Anspruch 6, **dadurch gekennzeichnet, dass** die wenigstens eine C₃-Vorläuferverbindung Propylen ist.

9. Verfahren nach Anspruch 6, **dadurch gekennzeichnet, dass** die wenigstens eine C₃-Vorläuferverbindung Propylen in Begleitung von Propan als Inertgasbestandteil ist.

10. Verfahren nach Anspruch 9, **dadurch gekennzeichnet, dass** das Propylen und das es begleitende Propan Bestandteil des Produktgasgemisch einer der Gasphasenpartialoxidation vorgeschalteten partiellen Dehydrierung und/oder Oxidehydrierung von Propan waren.

11. Verfahren nach einem der Ansprüche 1 bis 10, **dadurch gekennzeichnet, dass** die flüssige Phase P auf das Produktgasgemisch einer heterogen katalysierten Gasphasenpartialoxidation wenigstens einer C₃-Vorläuferverbindung der Acrylsäure zurückgeht und aus selbigem unter Anwendung wenigstens eines unscharfen Trennverfahrens erzeugt wurde.

12. Verfahren nach Anspruch 11, **dadurch gekennzeichnet, dass** das wenigstens eine unscharfe Trennverfahren wenigstens ein Trennverfahren aus der Gruppe enthaltend Absorption, partielle Kondensation, fraktionierende Kondensation Rektifikation, Strippung und Desorption umfasst.

13. Verfahren nach Anspruch 11 oder 12, **dadurch gekennzeichnet, dass** Methacrolein angereichert enthaltende Mutterlauge in wenigstens eines des wenigstens einen unscharfen Trennverfahrens rückgeführt wird.

14. Verfahren nach Anspruch 13, **dadurch gekennzeichnet, dass** Methacrolein angereichert enthaltende Mutterlauge in eine fraktionierende Kondensation des Produktgasgemischs der heterogen katalysierten Gasphasenpartialoxidation der wenigstens einen C₃-Vorläuferverbindung der Acrylsäure rückgeführt wird.

15. Verfahren nach einem der Ansprüche 1 bis 14, **dadurch gekennzeichnet, dass** die kristallisative Abtrennung mittels einer Suspensionskristallisation vorgenommen wird.

16. Verfahren nach Anspruch 15, **dadurch gekennzeichnet, dass** die Trennung von Suspensionskristallisat und verbliebener Mutterlauge mittels einer Waschkolonne vorgenommen wird.

17. Verfahren nach Anspruch 16, **dadurch gekennzeichnet dass** als Waschflüssigkeit die Schmelze von in der Waschkolonne vorab abgetrennten Acrylsäurekristallen verwendet wird.

18. Verfahren nach einem der Ansprüche 1 bis 17, **dadurch gekennzeichnet, dass** es die folgenden Verfahrensschritte umfasst:
a) Auskristallisieren von Acrylsäure aus der flüssigen Phase P;
b) Trennen des Acrylsäurekristallisats von der verbliebenen Mutterlauge;
c) wenigstens teilweises Aufschmelzen des abgetrennten Acrylsäurekristallisats und
d) wenigstens teilweises Rückführen des aufgeschmolzenen Acrylsäurekristallisats zum Schritt b) und/oder zum Schritt a).

19. Verfahren nach einem der Ansprüche 1 bis 18, **dadurch gekennzeichnet, dass** sich ein Verfahren, bei dem Acrylsäurekristallisat aufgeschmolzen und in Polymerisate radikalisch einpolymerisiert wird, anschließt.

## Claims

1. A process for removing methacrolein from liquid phase P comprising acrylic acid as a main constituent and target product, and methacrolein as a secondary component, wherein the liquid phase P, based on the amount of acrylic acid present therein, comprises from 0.01 to 15% by weight of methacrolein and the removal is effected by crystallization, the acrylic acid accumulating in the crystals formed and the methacrolein in the remaining mother liquor.

2. The process according to claim 1, wherein the liquid phase P comprises at least 10% by weight of acrylic acid.

3. The process according to claim 1 or 2, wherein the liquid phase P comprises at least 65% by weight of acrylic acid.

4. The process according to any of claims 1 to 3, wherein the liquid phase P comprises from 65 to 99.5% by weight of acrylic acid.

5. The process according to any of claims 1 to 4, wherein the liquid phase P, based on acrylic acid comprised therein, comprises from 0.2 to 30% by weight of water.

6. The process according to any of claims 1 to 5, wherein the liquid phase P stems from the product gas mixture of a heterogeneously catalyzed gas phase partial oxidation of at least one C₃ precursor compound of acrylic acid.

7. The process according to claim 6, wherein the at least one C₃ precursor compound is propane.

8. The process according to claim 6, wherein the at least one C₃ precursor compound is propylene.

9. The process according to claim 6, wherein the at least one C₃ precursor compound is propylene accompanied by propane as an inert gas constituent.

10. The process according to claim 9, wherein the propylene and the accompanying propane were a constituent of the product gas mixture of a partial dehydrogenation and/or oxydehydrogenation of propane which preceded the gas phase partial oxidation.

11. The process according to any of claims 1 to 10, wherein the liquid phase P stems from the product gas mixture of a heterogeneously catalyzed gas phase partial oxidation of at least one C₃ precursor compound of acrylic acid and has been obtained therefrom using at least one nonsharp separation process.

12. The process according to claim 11, wherein the at least one nonsharp separation process comprises at least one separation process from the group comprising absorption, partial condensation, fractional condensation, rectification, stripping and desorption.

13. The process according to claim 11 or 12, wherein mother liquor comprising accumulated methacrolein is recycled into at least one of the at least one nonsharp separation process.

14. The process according to claim 13, wherein mother liquor comprising accumulated methacrolein is recycled into a fractional condensation of the product gas mixture of the heterogeneously catalyzed gas phase partial oxidation of the at least one C₃ precursor compound of acrylic acid.

15. The process according to any of claims 1 to 14, wherein the crystallizative removal is undertaken by means of a suspension crystallization.

16. The process according to claim 15, wherein the separation of suspension crystals and remaining mother liquor is undertaken by means of a wash column.

17. The process according to claim 16, wherein the wash liquid used is the melt of acrylic acid crystals removed beforehand in the wash column.

18. The process according to any of claims 1 to 17, which comprises the following process steps:
a) crystallization of acrylic acid out of the liquid phase P;
b) separation of the acrylic acid crystals from the remaining mother liquor;
c) at least partial melting of the removed acrylic acid crystals and
d) at least partial recycling of the molten acrylic acid crystals to step b) and/or to step a).

19. The process according to any of claims 1 to 18, which is followed by a process in which acrylic acid crystals are melted and free-radically polymerized to polymers.

## Revendications

1. Procédé de séparation de méthacroléine d'une phase liquide P contenant de l'acide acrylique en tant que constituent principal et produit cible, et de la méthacroléine en tant que composant secondaire, **caractérisé en ce que** la phase liquide P contient, par rapport à la quantité d'acide acrylique qu'elle contient, 0,01 à 15 % en poids de méthacroléine, et la séparation a lieu par cristallisation, l'acide acrylique s'accumulant dans le cristallisat formé et la méthacroléine dans la liqueur mère restante.

2. Procédé selon la revendication 1, **caractérisé en ce que** la phase liquide P contient au moins 10 % en poids d'acide acrylique.

3. Procédé selon la revendication 1 ou 2, **caractérisé en ce que** la phase liquide P contient au moins 65 % en poids d'acide acrylique.

4. Procédé selon l'une quelconque des revendications 1 à 3, **caractérisé en ce que** la phase liquide P contient 65 à 99,5 % en poids d'acide acrylique.

5. Procédé selon l'une quelconque des revendications 1 à 4, **caractérisé en ce que** la phase liquide P contient, par rapport à l'acide acrylique qu'elle contient, 0,2 à 30 % en poids d'eau.

6. Procédé selon l'une quelconque des revendications 1 à 5, **caractérisé en ce que** la phase liquide P est dérivée du mélange gazeux de produits d'une oxydation partielle en phase gazeuse sous catalyse hétérogène d'au moins un composé précurseur en C₃ de l'acide acrylique.

7. Procédé selon la revendication 6, **caractérisé en ce que** ledit au moins un composé précurseur en C₃ est le propane.

8. Procédé selon la revendication 6, **caractérisé en ce que** ledit au moins un composé précurseur en C₃ est le propylène.

9. Procédé selon la revendication 6, **caractérisé en ce que** ledit au moins un composé précurseur en C₃ est le propylène accompagné de propane en tant que constituant gaz inerte.

10. Procédé selon la revendication 9, **caractérisé en ce que** le propylène et le propane l'accompagnant font partie du mélange gazeux de produits d'une déshydrogénation et/ou oxydéshydrogénation partielle de propane en amont de l'oxydation partielle en phase gazeuse.

11. Procédé selon l'une quelconque des revendications 1 à 10, **caractérisé en ce que** la phase liquide P est dérivée du mélange gazeux de produits d'une oxydation partielle en phase gazeuse sous catalyse hétérogène d'au moins un composé précurseur en C₃ de l'acide acrylique et a été formée à partir de celui-ci en utilisant au moins un procédé de séparation imprécis.

12. Procédé selon la revendication 11, **caractérisé en ce que** ledit au moins un procédé de séparation imprécis comprend au moins un procédé de séparation du groupe contenant l'absorption, la condensation partielle, la condensation fractionnée, la rectification, l'extraction et la désorption.

13. Procédé selon la revendication 11 ou 12, **caractérisé en ce que** la liqueur mère contenant la méthacroléine accumulée est recyclée dans au moins un dudit au moins un procédé de séparation imprécis.

14. Procédé selon la revendication 13, **caractérisé en ce que** la liqueur mère contenant la méthacroléine accumulée est recyclée dans une condensation fractionnée du mélange gazeux de produits de l'oxydation partielle en phase gazeuse sous catalyse hétérogène dudit au moins un composé précurseur en C₃ de l'acide acrylique.

15. Procédé selon l'une quelconque des revendications 1 à 14, **caractérisé en ce que** la séparation par cristallisation est réalisée au moyen d'une cristallisation en suspension.

16. Procédé selon la revendication 15, **caractérisé en ce que** la séparation du cristallisat en suspension et de la liqueur mère restante est réalisée au moyen d'une colonne de lavage.

17. Procédé selon la revendication 16, **caractérisé en ce que** la masse fondue des cristaux d'acide acrylique séparés auparavant dans la colonne de lavage est utilisée en tant que liquide de lavage.

18. Procédé selon l'une quelconque des revendications 1 à 17, **caractérisé en ce qu'**il comprend les étapes de procédé suivantes :
a) la cristallisation d'acide acrylique à partir de la phase liquide P ;
b) la séparation du cristallisat d'acide acrylique de la liqueur mère restante ;
c) la fusion au moins partielle du cristallisat d'acide acrylique séparé ; et
d) le recyclage au moins partiel du cristallisat d'acide acrylique fondu dans l'étape b) et/ou dans l'étape a).

19. Procédé selon l'une quelconque des revendications 1 à 18, **caractérisé en ce qu'**il est suivi par un procédé selon lequel le cristallisat d'acide acrylique est fondu et polymérisé par voie radicalaire en polymères.
